Europäisches Patentamt

European Patent Office

Office européen des brevets

(19)

(11) EP 0 796 103 B1

(12) EUROPÄISCHE PATENTSCHRIFT

(45) Veröffentlichungstag und Bekanntmachung des Hinweises auf die Patenterteilung:
**10.01.2001   Patentblatt 2001/02**

(51) Int Cl.⁷: **A61K 35/78**, A61K 31/19, A61K 31/215

(21) Anmeldenummer: **95940992.1**

(22) Anmeldetag: **21.11.1995**

(86) Internationale Anmeldenummer:
**PCT/EP95/04586**

(87) Internationale Veröffentlichungsnummer:
**WO 96/18407 (20.06.1996 Gazette 1996/28)**

(54) **VERWENDUNG VON WEIHRAUCH ZUR BEHANDLUNG DER ALZHEIMER-KRANKHEIT**

USE OF INCENSE IN THE TREATMENT OF ALZHEIMER'S DISEASE

UTILISATION DE L'ENCENS POUR LE TRAITEMENT DE LA MALADIE D'ALZHEIMER

(84) Benannte Vertragsstaaten:
**AT BE CH DE DK ES FR GB GR IE IT LI LU MC NL PT SE**

(30) Priorität: **13.12.1994   DE 4444288**

(43) Veröffentlichungstag der Anmeldung:
**24.09.1997   Patentblatt 1997/39**

(73) Patentinhaber: **Ayurmedica Arzneimittelforschung und -grosshandel GmbH & Co. Betriebs KG
82343 Pöcking (DE)**

(72) Erfinder: **Etzel,Rainer
82343 Pöcking (DE)**

(74) Vertreter:
**Weisert, Annekäte, Dipl.-Ing. Dr.-Ing. et al
Patentanwälte
Kraus & Weisert
Thomas-Wimmer-Ring 15
80539 München (DE)**

(56) Entgegenhaltungen:
**EP-A- 0 552 657          WO-A-90/01937**

- **LLOYDIA RESEARCH PAPER, Bd. 35, Nr. 3, 1972 Seiten 280-287, R. PERNET 'Phytochimie des Burseracees'**
- **J ECON TAXON BOT, Bd. 4, Nr. 3, 1983 Seiten 829-838, SINGH K K ET AL 'TRADITIONAL PHYTOTHERAPY AMONG THE TRIBALS OF VARANASI DISTRICT UTTAR PRADESH INDIA'**
- **IND. J. CHEM., Bd. 16B, Nr. 3, 1978 Seiten 176-178, R.S. PARDHY ET AL. 'Beta-Boswellic acid, acetyl-beta-boswellic acid, acetyl-11-keto-beta-boswellic acid & 11-keto-beta-boswellic acid, four pentacyclic triterpene acids from the resin of Boswellia serrata Roxb.' in der Anmeldung erwähnt**
- **DATABASE WPI Week 9515 Derwent Publications Ltd., London, GB; AN 95-107503 & CN,A,1 079 661 (ZHANG) , 22.Dezember 1993**
- **DATABASE WPI Week 9524 Derwent Publications Ltd., London, GB; AN 95-179423 & CN,A,1 082 886 (GUAN Z) , 2.März 1994**

Anmerkung: Innerhalb von neun Monaten nach der Bekanntmachung des Hinweises auf die Erteilung des europäischen Patents  kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 796 103 B1

**Beschreibung**

Die Erfindung betrifft die Verwendung von Weihrauch (Olibanum), Weihrauchextrakten, in Weihrauch enthaltenen Substanzen, ihren physiologisch annehmbaren Salzen, ihren Derivaten und deren physiologischen Salzen, reiner Boswelliasäure, eines physiologisch annehmbaren Salzes, eines Derivats, eines Salzes des Derivats, zur Herstellung eines Arzneimittels zur prophylaktischen und therapeutischen Behandlung der Alzheimer-Krankheit (Morbus Alzheimer).

Die Alzheimer-Krankheit ist eine etwa ab dem fünften Lebensjahrzehnt auftretende Degenerationskrankheit mit morphologischen und biochemischen Veränderungen von Gehirnarealen besonders im Bereich des Hippocampus und des Assoziationskortex. Derzeit wird eine deutliche Zunahme der Erkrankungshäufigkeit beobachtet. Die Symptome dieser Krankheit sind Desorientiertheit mit Störungen cognitiver Fähigkeiten, Gedächtnisschwund und Wesensveränderungen mit emotionaler Instabilität, totale Regression bis zur völligen Pflegebedürftigkeit und Demenz.

In Deutschland leiden etwa 850.000 Menschen an der Alzheimer-Krankheit, weltweit dürften ca. 15 Millionen Menschen von dieser Krankheit betroffen sein.

Die therapeutischen Möglichkeiten für die Behandlung dieser Krankheit sind bisher unbefriedigend. Da die Ursachen für die Entstehung der Alzheimer-Krankheit noch nicht bekannt sind, stehen keine gezielt wirkenden Therapeutika zur Verfügung. Ebenfalls fehlt es an Präparaten, die zumindest die Symptome der Alzheimer-Krankheit lindern könnten.

Die bisher bekannten Therapieansätze sind nicht zufriedenstellend und ohne klinische Relevanz. Als einziges derzeit zugelassenes Medikament ist Tacrine mit geringer Ansprechrate, unbefriedigenden Therapieergebnissen und deutlichen, vor allem hepatotoxischen Nebenwirkungen als Langzeittherapie noch wenig zufriedenstellend. Versuche mit Monosialogangliosiden als Wachstumsfaktoren-Stimulation der Nervenzellen blieben ohne signifikante Wirkung.

Der Erfindung liegt daher die Aufgabe zugrunde, die Verwendung von Präparaten zur Verfügung zu stellen, die der Behandlung der Alzheimer-Krankheit dienen. Das erfindungsgemäß zur Verfügung gestellte Präparat soll des weiteren in der Lage sein, das Entstehen der Alzheimer-Krankheit zu verhindern oder hinauszuzögern. Das erfindungsgemäß zur Verfügung gestellte Arzneimittel soll untoxisch sein und von den Patienten gut toleriert werden.

Überraschenderweise wurde jetzt gefunden, daß Weihrauch (Olibanum), Weihrauchextrakte, in Weihrauch enthaltene Substanzen, ihre physiologisch annehmbaren Salze, ihre Derivate und deren physiologische Salze, reine Boswelliasäure, ein physiologisch annehmbares Salz, ein Derivat, ein Salz des Derivats äußerst wirksam für die Behandlung der Alzheimer-Krankheit sind.

In der ayurvedischen Medizin Indiens werden Arzneimittel, die Präparationen aus der Pflanze Boswellia serrata enthalten, zur Behandlung von Entzündungen, aber auch Rheumatismus verwendet. Hinweise, diese Arzneimittel auch zur Behandlung der Alzheimer-Krankheit einzusetzen, finden sich in der Literatur nicht. Aufgrund der biologischen Aktivität der Inhaltsstoffe von Boswellia serrata hat man versucht, ihre Struktur aufzuklären. So berichten Pardhy & Bhattacharyya in Ind. J. Chem., 16B:176-178, 1978, daß Boswellia serrata im wesentlichen die folgenden Inhaltsstoffe enthält:

β-Boswelliasäure, Acetyl-β-boswelliasäure, Acetyl-11-keto-β-boswelliasäure, 11-Keto-β-boswelliasäure.

Pharmakologische Untersuchungen über die prophylaktische und/oder therapeutische Wirksamkeit der genannten Boswelliasäuren sind in Bezug auf die Alzheimer-Krankheit in der Literatur nicht beschrieben.

Als Boswelliasäure wird vorzugsweise β-Boswelliasäure verwendet, die nach Literaturangaben aus Boswellia serrata oder anderen bekannten Boswelliasäure enthaltenden Pflanzen isoliert wird. Die β-Boswelliasäure kann geringe Mengen an α-oder γ-Boswelliasäure enthalten. Als physiologisch annehmbare Salze der Boswelliasäure können die Natrium-, Kalium-, Ammonium-, Calciumsalze verwendet werden. Als Derivate der Boswelliasäure können niedere Alkylester, die durch Veresterung der Carboxylgruppe mit einem $C_1$-$C_6$-Alkohol erhalten werden, vorzugsweise der Methylester, oder Ester, die durch Veresterung der Hydroxylgruppe mit einer physiologisch verträglichen Carbonsäure erhalten werden, verwendet werden. Bevorzugte Derivate sind β-Boswelliasäureacetat, β-Boswelliasäureformiat, β-Boswelliasäuremethylester, Acetyl-β-boswelliasäure, Acetyl-11-keto-β-boswelliasäure und 11-Keto-β-boswelliasäure.

Erfindungsgemäß ist es weiterhin möglich, eine Boswelliasäure enthaltende pflanzliche Zubereitung zu verwenden. Erfindungsgemäß ist es bevorzugt, Präparate, die aus Weihrauch bzw. Weihrauchharzen gewonnen werden, zu verwenden.

Pflanzen, die Boswelliasäure (syn.: Boswellinsäure) enthalten, sind Weihrauchpflanzen (Boswellia-Arten), insbesondere:

Boswellia (serrata, papyrifera, frereana, carteri, thurifera, glabra, bhaw-dajiana, oblongata, socotrana und andere Vertreter dieser Familie).

Eine besonders bevorzugte Boswelliasäure enthaltende pflanzliche Zubereitung ist das von der Firma Ayurmedica, Pöcking, vertriebene Phytopharmakon H 15, ein liphophiler Extrakt aus Boswellia serrata. Dieses verschreibungspflichtige Arzneimittel enthält als Wirkstoff einen Trockenextrakt aus Olibanum. Die Handelsprodukte Tablette bzw. Granulat setzen sich wie folgt zusammen:

1 Tablette enthält 400 mg Trockenextrakt aus Olibanum (4,2 - 5,9:1), Auszugsmittel: Chloroform/Methanol

1 g Granulat enthält 500 mg Trockenextrakt aus Olibanum (4,2 - 5,9:1), Auszugsmittel: Chloroform/Methanol.

Erfindungsgemäß können natürliche und synthetische Verbindungen verwendet werden.

Erfindungsgemäß ist es weiterhin möglich, daß die Verwendung zusammen mit anderen chemisch reinen Arzneistoffen und/oder anderen pflanzlichen Arzneimitteln erfolgt.

Erfindungsgemäß werden die Zubereitungen je nach Bedarf verabreicht. Da die Zubereitungen, die Boswelliasäuren, ihre Salze und Derivate enthalten, wenig toxisch sind, ist die Dosierung nicht kritisch und kann in Abhängigkeit von der Schwere der Krankheit, dem Gewicht des zu behandelnden Patienten, dem Verabreichungsweg, der Häufigkeit der Verabreichung und der Dauer der Behandlung vom Arzt leicht variiert werden.

Einheitsdosen können beispielsweise ein- bis viermal täglich verabreicht werden. Die exakte Dosis hängt vom Verabreichungsweg und.dem zu behandelnden Zustand ab. Naturgemäß kann es erforderlich sein, Routinevariationen der Dosis, je nach dem Alter und dem Gewicht des Patienten sowie der Schwere des zu behandelnden Krankheitszustandes, vorzunehmen.

Die erfindungsgemäß verwendeten Zubereitungen können in an sich bekannter Weise unter Verwendung eines oder mehrerer pharmazeutisch annehmbarer Träger oder Verdünnungsmittel formuliert werden. Die Zubereitungen können für die orale, parenterale, rektale oder intranasale Verabreichung oder in einer für die Verabreichung durch Inhalation oder Insufflation geeigneten Weise formuliert werden. Zubereitungen der Verbindungen für die orale Verabreichung sind bevorzugt.

Die pharmazeutischen Zubereitungen für die orale Verabreichung können in Form von beispielsweise Tabletten oder Kapseln, die nach an sich bekannten Verfahren mit pharmazeutisch annehmbaren Verdünnungsmitteln, wie Bindemitteln (zum Beispiel vorgelatinierter Maisstärke, Polyvinylpyrrolidon oder Hydroxypropylmethylcellulose), Füllstoffen (zum Beispiel Lactose, Saccharose, Mannit, Maisstärke, mikrokristalline Cellulose oder Calciumhydrogenphosphat); Schmiermitteln (zum Beispiel Stearinsäure, Polyethylenglykol, Magnesiumstearat, Talk oder Siliciumdioxid); Desintegrationsmitteln (zum Beispiel Kartoffelstärke, Natriumstärkeglykolat oder Natriumcarboxymethylcellulose); oder Benetzungsmitteln (zum Beispiel Natriumlaurylsulfat), hergestellt werden, vorliegen. Die Tabletten können nach an sich bekannten Verfahren überzogen werden.

Flüssige Zubereitungen für die orale Verabreichung können in Form von beispielsweise wäßrigen oder öligen Lösungen, Sirupen, Elixieren, Emulsionen oder Suspensionen vorliegen, oder sie können als Trockenprodukt für die Konstitution mit Wasser oder einem anderen geeigneten Träger vor der Verwendung vorliegen. Solche flüssigen Zubereitungen können nach an sich bekannten Verfahren mit pharmazeutisch annehmbaren Zusatzstoffen hergestellt werden, wie Suspensionsmitteln (zum Beispiel Sorbitsirup, Cellulosederivaten, Glucose/Zucker-Sirup, Gelatine, Aluminiumstearatgel oder hydrierten genießbaren Fetten); Emulgiermitteln (zum Beispiel Lecithin, Gummi arabicum oder Sorbitanmonooleat); nichtwäßrigen Trägern (zum Beispiel Mandelöl, öligen Estern, Ethylalkohol oder fraktionierten Pflanzenölen); und Konservierungsmitteln (zum Beispiel Methyl- oder Propyl-p-hydroxybenzoaten oder Sorbinsäure). Die flüssigen Zubereitungen können auch an sich bekannte Puffer, Geschmacks- bzw. Aromamittel, Farbstoffe und Süßstoffe, je nach Bedarf, enthalten.

Für die parenterale Verabreichung können die Zubereitungen für Injektion, bevorzugt für die intravenöse, intramuskuläre oder für subkutane Injektion, formuliert werden. Zubereitungen für die Injektion können in Eindosenform, zum Beispiel in Ampullen, oder in Mehrfachdosis-Behältern mit einem zugegebenen Konservierungsstoff vorliegen. Die Zubereitungen können in Form von Suspensionen, Lösungen oder Emulsionen in öligen oder wäßrigen Trägern vorliegen und Zubereitungshilfsmittel enthalten, wie Suspendier-, Stabilisier- und/oder Dispersionsmittel, und/oder Mittel zur Einstellung der Tonizität der Lösung enthalten. Alternativ kann der aktive Bestandteil in Pulverform für die Konstitution mit einem geeigneten Träger, zum Beispiel sterilem pyrogenfreien Wasser, vor der Verwendung vorliegen.

Die Verbindungen können ebenfalls als rektale Zubereitungen, wie Suppositorien, formuliert werden, zum Beispiel solche, die an sich bekannte Suppositorien-Grundstoffe, wie Kakaobutter oder andere Glyceride, enthalten.

Für die intranasale Verabreichung können die Verbindungen als flüssige Sprays, in Form von Tropfen oder als Schnupfpulver verwendet werden.

Für die Verabreichung durch Inhalation werden die Verbindungen zweckdienlich in Form eines Aerosolsprays aus einer unter Druck stehenden Packung unter Verwendung geeigneter Treibmittel oder in einer Zerstäubungsvorrichtung abgegeben. Im Falle eines unter Druck stehenden Aerosols wird die Dosiseinheit bestimmt, indem ein Ventil vorgesehen wird, welches eine abgemessene Menge freigibt. Kapseln und Patronen aus beispielsweise Gelatine für die Verwendung in einer Inhalationsvorrichtung oder einer Insufflationsvorrichtung können so zubereitet werden, daß sie ein Pulvergemisch aus einer erfindungsgemäß verwendeten Verbindung und einem geeigneten Pulver-Grundstoff, wie Lactose oder Stärke, enthalten.

Die folgenden Beispiele erläutern die erfindungsgemäße Verwendung.

**Beispiel 1**

| H 15 Ayurmedica | |
|---|---|
| H 15-Tabletten zu 400 mg | |
| | |
| Boswellia-serrata-Extrakt | 41,0 kg |
| sterilisierte Lactose | 14,0 kg |
| sterilisiertes Aerosil | 2,0 kg |
| sterilisiertes M.C.C.P. | 4,0 kg |
| sterilisierte Stärke | 3,0 kg |
| sterilisiertes Magnesiumstearat | 1,0 kg |
| sterilisierter Talk | 2,0 kg |
| sterilisiertes Natriumstärkeglykolat | 1,0 k |
| insgesamt: | 68,0 kg |

Der Boswellia-serrata-Extrakt wird mit den übrigen Zutaten vermischt und in an sich bekannter Weise zu Tabletten verpreßt.

**Beispiel 2**

Tabletten für die orale Verabreichung

A. Direkte Kompression

1)

| a) verschiedene Extrakte von Weihrauch (Olibanum) | |
|---|---|
| b) pulverisierte Droge | 0,5 - 1,0 g/Tablette bzw. |
| c) Wirkstoff: Boswelliasäure | 15 - 30 mg/Tablette |
| Magnesiumstearat BP | 0,65 mg/Tablette |
| wasserfreie Lactose | 80 mg/Tablette |

Der Wirkstoff wird mit der wasserfreien Lactose und dem Magnesiumstearat vermischt, und das Gemisch wird gesiebt. Das entstehende Gemisch wird zu Tabletten unter Verwendung einer Tablettiermaschine verpreßt.

2)

| a) verschiedene Extrakte von Weihrauch (Olibanum) | |
|---|---|
| b) pulverisierte Droge | 0,5 - 1,0 g/Tablette bzw. |
| c) Wirkstoff: Boswelliasäure | 15 - 30 mg/Tablette |
| Magnesiumstearat BP | 0,7 mg/Tablette |
| mikrokristalline Cellulose NF | 100 mg/Tablette |

Der Wirkstoff wird gesiebt und mit der mikrokristallinen Cellulose und dem Magnesiumstearat vermischt. Das entstehende Gemisch wird unter Verwendung einer Tablettiermaschine zu Tabletten verpreßt.

B. Nasse Granulierung

| a) verschiedene Extrakte von Weihrauch (Olibanum) | |
|---|---|
| b) pulverisierte Droge | 0,5 - 1,0 g/Tablette bzw. |
| c) Wirkstoff: Boswelliasäure | 15 - 30 mg/Tablette |
| Lactose BP | 150,0 mg/Tablette |
| Stärke BP | 30,0 mg/Tablette |
| vorgelatinierte Maisstärke BP | 15,0 mg/Tablette |
| Magnesiumstearat BP | 1,5 mg/Tablette |

Der Wirkstoff wird durch ein geeignetes Sieb gesiebt und mit der Lactose, der Stärke und der vorgelatinierten Maisstärke vermischt. Geeignete Volumina an gereinigtem Wasser werden zugegeben, und das Pulver wird granuliert. Nach dem Trocknen wird das Granulat gesiebt und mit dem Magnesiumstearat vermischt. Das Granulat wird dann unter Verwendung von Lochstanzen mit geeignetem Durchmesser zu Tabletten verpreßt.
Tabletten anderer Zusammensetzung können hergestellt werden, indem man das Verhältnis von Wirkstoff zu Lactose oder das Kompressionsgewicht ändert und entsprechende Lochstanzen verwendet.

**Beispiel 3**

| Kapseln | |
|---|---|
| a) verschiedene Extrakte von Weihrauch (Olibanum) | |
| b) granulierte Droge | 0,5 - 1,0 g/Tablette bzw. |
| c) Wirkstoff: Boswelliasäure | 15 - 30 mg/Tablette |
| freifließende Stärke | 150,00 mg/Kapsel |
| Magnesiumstearat BP | 1,00 mg/Kapsel |

Der Wirkstoff wird gesiebt und mit den anderen Bestandteilen vermischt. Das Gemisch wird unter Verwendung einer geeigneten Vorrichtung in Hartgelatinekapseln Nr. 2 gefüllt. Andere Kapseln können hergestellt werden, indem man das Füllgewicht ändert und erforderlichenfalls die Kapselgröße entsprechend ändert.

**Beispiel 4**

<u>Sirup</u>

| Saccharose-freie Zubereitung | | mg/5 ml Dosis |
|---|---|---|
| a) verschiedene Extrakte von Weihrauch (Olibanum) | | |
| b) granulierte Droge bzw. | | |
| c) Wirkstoff: Boswelliasäure | | 15 - 30 |
| Hydroxypropylmethylcellulose USP (Viskositäts-Typ 4000) | | 22,5 |
| Puffer | ) | |
| Geschmacksstoff | ) | |
| Farbstoff | ) | nach Bedarf |
| Konservierungsmittel | ) | |
| Süßstoff | ) | |
| gereinigtes Wasser | auf | 5,0 ml |

Die Hydroxypropylmethylcellulose wird in heißem Wasser dispergiert, abgekühlt und dann mit einer wäßrigen Suspension vermischt, die den Wirkstoff und die anderen Komponenten der Zubereitung enthält. Die entstehende Lösung wird auf ihr Volumen eingestellt und vermischt.

**Beispiel 5**

| Suspension | | mg/5 ml Dosis |
|---|---|---|
| a) getrockneter Drogenextrakt von Weihrauch (Olibanum) | | |
| b) pulverisierte Droge | | 0,5 - 1,0  bzw. |
| c) Wirkstoff: Boswelliasäure | | 15 - 30 |

```
Aluminiummonostearat                              75,00

Süßstoff                )

Geschmacksstoff         )                    nach Bedarf

Farbstoff               )

fraktioniertes  Kokosnußöl    auf            5,00
```

Das Aluminiummonostearat wird in etwa 90% des fraktionierten Kokosnußöls dispergiert. Die resultierende Suspension wird unter Rühren auf 115°C erhitzt und dann abgekühlt. Die Süß-, Geschmacks- und Farbstoffe werden zugesetzt, und der Wirkstoff wird dispergiert. Die Suspension wird mit dem restlichen fraktionierten Kokosnußöl auf das Volumen eingestellt und vermischt.

**Beispiel 6**

| Sublinguale Tablette | |
|---|---|
| a) Drogenextrakt von Weihrauch (Olibanum) | 0,5 - 1,0 g/Tablette |
| b) Wirkstoff: Boswelliasäure | 15 - 30 mg/Tablette bzw. |
| c) pulverisierte Droge | |
| verpreßbarer Zucker NF | 50,5 mg/Tablette |
| Magnesiumstearat BP | 0,5 mg/Tablette |

Der Wirkstoff wird durch ein geeignetes Sieb gesiebt, mit den anderen Bestandteilen vermischt und unter Verwendung geeigneter Lochstanzen verpreßt. Tabletten anderer Stärke können hergestellt werden, indem man das Verhältnis von Wirkstoff zu Träger oder das Kompressionsgewicht ändert.

**Beispiel 7**

| Suppositorien für die rektale Verabreichung | |
|---|---|
| a) Extrakte von Weihrauch (Olibanum) | |
| b) Wirkstoff: Boswelliasäure | 15 - 30 mg bzw. |
| c) Witepsol H15[+] auf | 1,0 g |

[+] geeignete Qualität von Adeps solidus Ph.Eur.

Eine Suspension des Wirkstoffs in geschmolzenem Witepsol wird hergestellt und unter Verwendung einer geeigneten Vorrichtung in 1-g-Suppositorienformen eingefüllt.

**Beispiel 8**

| Injektion für intravenöse Verabreichung | | |
|---|---|---|
| a) Extrakte von Weihrauch (Olibanum) bzw. | | |
| b) Wirkstoff: Boswelliasäure | | 15 - 30 mg/ml |
| Natriumchlorid-intravenöse Infusion, BP, 0,9% Gew./Vol. | auf | 1 ml |

(fortgesetzt)

| Injektion für intravenöse Verabreichung | | |
|---|---|---|
| Ansatzgröße | 2500 ml | |

Der Wirkstoff wird in einem Teil der Natriumchlorid-intravenösen Infusion gelöst, die Lösung mit der Natriumchloridintravenösen Infusion auf das Volumen eingestellt und die Lösung gründlich vermischt. Die Lösung wird in klare, Typ 1, 10-ml-Glasampullen eingefüllt und unter Stickstoff im Kopfraum durch Abschmelzen des Glases abgesiegelt. Die Ampullen werden durch Erhitzen im Autoklaven bei 120°C für nicht kürzer als 20 Minuten sterilisiert.

**Beispiel 9**

| Patrone für die Inhalation | |
|---|---|
| a) Extrakte von Weihrauch (Olibanum) bzw. b) Wirkstoff (mikronisiert): Boswelliasäure Lactose BP | 15 - 30 mg/Patrone 25,00 |

Der Wirkstoff wird in einer Strahlmühle zu einem feinen Teilchengrößenbereich mikronisiert und dann mit der Lactose vermischt. Die Pulvermischung wird in Hartgelatinekapseln Nr. 3 eingefüllt.

**Beispiel 10**

```
Nasenspray
a) Extrakte von Weihrauch (Olibanum) bzw.
b) Wirkstoff: Boswelliasäure      1,5 - 3,0 %/Vol.
Konservierungsmittel         )    nach Bedarf
Natriumchlorid BP            )
gereinigtes Wasser BP       auf  100


Abgabegewicht                    100 mg (Äquivalent zu
                                 7 mg Wirkstoff)
```

Der Wirkstoff, der Konservierungsstoff und das Natriumchlorid werden in einem Teil des Wassers gelöst. Die Lösung wird mit Wasser auf das Volumen eingestellt und die Lösung gründlich vermischt.

Fallbeispiele

Für die Therapie wird das genannte Phytopharmakon H 15 vorzugsweise in Form von Tabletten verabreicht. Bei einer Höchstdosis von 3 x 2 Tabletten pro Tag wird in der Regel 3 x 1 Tablette pro Tag jeweils nach dem Essen über längere Zeit von dem Patienten eingenommen.
Nach drei bis sechs Wochen ist eine deutliche Verbesserung des klinischen Gesamtbildes der behandelten Patienten zu verzeichnen.
Für die prophylaktische Anwendung werden 2 x 1 Tablette bis 3 x 1 Tablette nach dem Essen pro Tag über mehrere Monate hinweg zur Dauerprävention verabreicht.
Die Wirksamkeit der Therapie wird durch die folgenden Fallbeispiele veranschaulicht:

**Fallbeispiel 1**

Eine 83jährige Patientin, die seit zwei Jahren die Symptome der Alzheimer-Krankheit zeigt (Gedächtnisreduktion, Probleme mit der Wortfindung, kognitive Minderfunktion, Aggression), erhält einen Monat lang 3 x 1 Tablette des Phytopharmakons H 15 pro Tag. Vier Wochen nach Therapiebeginn ist eine beginnende Aufhellung und deutliche Reduzierung der "Verwirrtheit" der Patientin zu beobachten. Eine über mehrere Monate erfolgende Dauertherapie (2 x 1 Tablette H 15 pro Tag) führt zu einer deutlichen und konstanten Verbesserung des gesamtklinischen Befundbildes, was auch von den die Patienten umgebenden Familienangehörigen bestätigt wird.

**Fallbeispiel 2**

Ein 81jähriger Alzheimer-Patient wird über einen Zeitraum von drei Wochen mit einer Dosierung von 3 x 2 Tabletten H 15 täglich behandelt. Anschließend wird die Dosierung halbiert (3 x 1 Tablette H 15 täglich). Die Wortfindungsstörungen, die Desorientiertheit und das Defizit bei der kognitiven Fähigkeit wird ab der vierten Woche nach Therapiebeginn deutlich besser. Mit der Reduzierung der Therapie auf 3 x 1 Tablette scheint zunächst ein gewisser Stillstand für ca. vier Wochen einzutreten, dann aber eine weitere Verbesserung aller Defizite.

**Fallbeispiel 3**

Ein 79jähriger Alzheimer-Patient mit wechselnden Symptomen (vor allem Orientierungsstörungen, Verkennung der Realität, starke Stimmungsschwankungen mit Aggression) erhält 3 x 1 Tablette H 15 pro Tag.
Die Orientierungsstörungen lassen nach drei Wochen deutlich nach, und der Patient erkennt seine ihn umgebenden Verwandten wieder zunehmend regelmäßig als solche. Seine Stimmung wird zunehmend stabiler, die aggressiven Ausbrüche lassen nach, und seine Sprache wird geordneter.

**Fallbeispiel 4**

Einer 63jährigen Patientin, die an der Alzheimer-Krankheit in bereits fortgeschrittenem Stadium leidet (die Diagnose wurde vor vier Jahren gestellt; die Patientin erkennt ihre Kinder nicht mehr und ist bezüglich höherer menschlicher Kontroll- und Regel funktionen wie Stuhlgang und Ernährung völlig desorientiert und dekompensiert), werden 3 x 1 Tablette H 15 täglich verabreicht. Nach drei Wochen ist eine erste erkennbare Verbesserung ihrer Globalsituation zu verzeichnen, die zwar langsam, aber stetig fortschreitet.

**Fallbeispiel 5**

Eine 59 jährige Alzheimer-Patientin, die seit fünf Jahren an dieser Krankheit leidet und die entsprechenden Symptome zeigt, ist desorientiert, erkennt ihre Umgebung und Verwandten nicht mehr und ist deshalb zum Pflegefall geworden. Eine Behandlung mit 3 x 2 Tabletten H 15 täglich führt zu einer Verbesserung ihres Krankheitsbildes nach vier Wochen.

**Patentansprüche**

1. Verwendung von Weihrauch (Olibanum), Weihrauchextrakten, in Weihrauch enthaltenen Substanzen, ihren physiologisch annehmbaren Salzen, ihren Derivaten und deren physiologischen Salzen, reiner Boswelliasäure, eines physiologisch annehmbaren Salzes, eines Derivats, eines Salzes des Derivats, zur Herstellung eines Arzneimittels zur prophylaktischen und therapeutischen Behandlung der Alzheimer-Krankheit.

2. Verwendung nach Anspruch 1, dadurch **gekennzeichnet**, daß man reine Boswelliasäure, ein physiologisch annehmbares Salz, ein Derivat, ein Salz des Derivats, einen Boswelliasäure enthaltenden Weihrauchextrakt für die Herstellung des Arzneimittels einsetzt.

3. Verwendung nach einem der Ansprüche 1 oder 2, dadurch **gekennzeichnet**, daß man einen Extrakt aus dem Harz der Boswellia serrata zur Herstellung des Arzneimittels einsetzt.

4. Verwendung nach einem der Ansprüche 1 bis 3, dadurch **gekennzeichnet**, daß die Verwendung zur Herstellung eines Arzneimittels für die intraperitoneale, orale, bukkale, rektale, intramuskuläre, topische, subkutane, intraartikuläre oder intravenöse Verabreichung erfolgt.

**5.** Verwendung nach einem der Ansprüche 1 bis 3, dadurch **gekennzeichnet,** daß die Verwendung zur Herstellung eines Arzneimittels in Form von Tabletten, Dragees, Kapseln, Injektionslösungen, Lösungen, Emulsionen, Salben, Cremes, Inhalationsapparaten, Aerosolen oder Suppositorien erfolgt.

## Claims

**1.** The use of frankincense (olibanum), frankincense extract, substances contained in frankincense, physiologically compatible salts thereof, derivatives thereof and physiological salts of said derivatives, of pure boswellic acid, of a physiologically compatible salt, or of a derivative or a salt of said derivative, for the production of a drug for the prophylactic and therapeutic treatment of Alzheimer's disease.

**2.** A use according to claim 1, characterised in that pure boswellic acid, a physiologically compatible salt, a derivative, a salt of said derivative or a frankincense extract which contains boswellic acid, is used for the production of said drug.

**3.** A use according to either one of claims 1 or 2, characterised in that an extract of the resin of *Boswellia serrata* is used for the production of said drug.

**4.** A use according to any one of claims 1 to 3, characterised in that said use is implemented for the production of a drag for intraperitoneal, oral, buccal, rectal, intramuscular, topical, subcutaneous, intra-arterial or intravenous administration.

**5.** A use according to any one of claims 1 to 3, characterised in that said use is implemented for the production of a drug in the form of tablets, dragées, capsules, injection solutions, solutions, emulsions, ointments, creams, inhalation apparatuses, aerosols or suppositories.

## Revendications

**1.** Utilisation d'encens (oliban), d'extraits d'encens, de substances contenues dans de l'encens, de leurs sels pouvant être acceptés physiologiquement, de leurs dérivés et de leurs sels physiologiques, d'acide de Boswellia pur, d'un sel pouvant être accepté physiologiquement, d'un dérivé, d'un sel du dérivé, pour la fabrication d'un médicament destiné au traitement prophylactique et thérapeutique de la maladie d'Alzheimer.

**2.** Utilisation selon la revendication 1, caractérisée en ce qu'on utilise de l'acide de Boswellia pur, un sel pouvant être accepté physiologiquement, un dérivé, un sel du dérivé, un extrait contenant de l'acide de Boswellia, pour la fabrication du médicament.

**3.** Utilisation selon l'une des revendications 1 ou 2, caractérisée en ce qu'on utilise un extrait de la résine du Boswellia serrata pour la fabrication du médicament.

**4.** Utilisation selon l'une des revendications 1 à 3, caractérisée en ce que l'utilisation s'effectue pour la fabrication d'un médicament en vue d'une administration intrapéritonéale, orale, buccale, rectale, intramusculaire, topique, sous-cutanée, intra-articulaire ou intraveineuse.

**5.** Utilisation selon l'une des revendications 1 à 3, caractérisée en ce que l'utilisation pour la fabrication d'un médicament s'effectue sous forme de tablettes, de dragées, de gélules, de solutions pour injection, de solutions, d'émulsions, de pommades, de crèmes, d'appareils à inhalation, d'aérosols ou de suppositoires.